(19) **Europäisches Patentamt**
**European Patent Office**
**Office européen des brevets**

(11) **EP 3 544 515 B1**

(12) **EUROPEAN PATENT SPECIFICATION**

(45) Date of publication and mention
of the grant of the patent:
**06.01.2021 Bulletin 2021/01**

(51) Int Cl.:
**B06B 1/02** (2006.01)     **H01L 41/04** (2006.01)

(21) Application number: **17809221.9**

(22) Date of filing: **20.11.2017**

(86) International application number:
**PCT/EP2017/079702**

(87) International publication number:
**WO 2018/095833 (31.05.2018 Gazette 2018/22)**

(54) **ULTRASOUND DEVICE AND ACOUSTIC COMPONENT FOR USE IN SUCH A DEVICE**

ULTRASCHALLVORRICHTUNG UND AKUSTISCHES BAUELEMENT ZUR VERWENDUNG IN SOLCH EINER VORRICHTUNG

DISPOSITIF À ULTRASONS ET COMPOSANT ACOUSTIQUE DESTINÉ À ÊTRE UTILISÉ DANS UN TEL DISPOSITIF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.11.2016 EP 16199933**

(43) Date of publication of application:
**02.10.2019 Bulletin 2019/40**

(73) Proprietor: **Koninklijke Philips N.V.**
**5656 AG Eindhoven (NL)**

(72) Inventors:
• **JOHNSON, Mark, Thomas**
  **5656 AE Eindhoven (NL)**
• **HAKKENS, Franciscus, Johannes, Gerardus**
  **5656 AE Eindhoven (NL)**
• **VAN DE MOLENGRAAF, Roland, Alexander**
  **5656 AE Eindhoven (NL)**
• **VAN DEN ENDE, Daan, Anton**
  **5656 AE Eindhoven (NL)**

(74) Representative: **Marsman, Albert Willem**
**Philips Intellectual Property & Standards**
**High Tech Campus 5**
**5656 AE Eindhoven (NL)**

(56) References cited:
**US-A- 4 580 451     US-A- 5 638 822**

**Description**

FIELD OF THE INVENTION

The present invention relates to an ultrasound device comprising a transducer arrangement for on-body or in-body ultrasound treatment or imaging. It also relates to an acoustic component for use in such a device.

BACKGROUND OF THE INVENTION

US5638822 discloses an ultrasonic probe for coupling acoustic signals between the probe and a medium is provided. The ultrasonic probe has a piezoelectric element having a plurality of piezoelectric layers each having a different acoustic impedance. The piezoelectric layers are stacked in progressive order of acoustic impedance such that the layer with the acoustic impedance nearest to that of the medium is proximate the medium. At least one of said piezoelectric layers is made of piezoelectric composite material. The ultrasonic probe further has an electrode means for electrically coupling the piezoelectric layers to a voltage source for applying an oscillation voltage potential to each piezoelectric layer. The probe further has a control means for controlling the polarization of at least one of the piezoelectric layers. Document US5638822 may be considered to disclose an acoustic coupling component for positioning between an ultrasound transducer arrangement and material to be treated or imaged, the acoustic coupling component comprising: an electroactive material actuator comprising a layered electroactive material resonance structure of at least one electroactive material layer, and a further material layer of different acoustic impedance, wherein the electroactive material actuator is adapted to receive an ultrasound signal having a frequency from the ultrasound transducer arrangement, and to transmit the ultrasound signal to the material to be treated or imaged; and a controller for electrically controlling the electroactive material actuator to implement a control of the magnitude and direction of the stress vectors in each layer separately, through actuation of the at least one electroactive material layer, to implement a selection of the proper frequency response for the desired application for transmission of the ultrasound signal to the material to be treated or imaged.

Ultrasound waves find several applications in medicine. One such application is ultrasound imaging, wherein ultrasound waves are emitted by an ultrasound device comprising an array of ultrasound transducers into the body of a patient and echoes of the ultrasound waves are collected by the ultrasound transducers or by dedicated ultrasound receivers and processed to generate an ultrasound image, e.g. a ID, 2D or 3D ultrasound image. Another application is ultrasound therapy such as high intensity focused ultrasound (HIFU) therapy in which ultrasound beams are generated by an ultrasound device comprising ultrasound transducer element tiles and are focused on diseased tissue. The significant energy deposition at the focus creates local temperatures in the range of about 65°C to 85°C, which destroys the diseased tissue by coagulative necrosis.

To create good quality signal transfer between the ultrasound transducer and the body tissue being imaged or treated, good quality acoustic coupling is required. For example, special gels may be used that improve the contact between the ultrasound transducer array and the body tissue. This avoids transducer to air, and air to body interfaces.

An output window of an ultrasound transducer typically has an acoustic impedance which is designed based on the nature of the body tissue and the ultrasound frequency to be used.

Such an output window is thus designed for a particular imaging or treatment modality. However, some treatments and imaging procedures involve multiple processes, for example at different frequencies. For example, resonance imaging and harmonic imaging may be performed at different frequencies, and different treatment frequencies may be desired.

SUMMARY OF THE INVENTION

It would be desirable to enable setting an acoustic impedance of an acoustic coupling component, and enabling actuation of the component so that the ultrasound system can be optimized for different use cases.

The invention is defined by the independent claims. The dependent claims define advantageous embodiments.

Examples in accordance with an aspect of the invention provide an acoustic coupling component for positioning between an ultrasound transducer arrangement and material (such as a tissue) to be treated or imaged, comprising:

an electroactive material actuator comprising a layered electroactive material resonance structure of at least one electroactive material layer with adjustable thickness, and a further material layer of different acoustic impedance, wherein the electroactive material actuator is adapted to receive an ultrasound signal having a frequency from the ultrasound transducer arrangement, and to transmit the ultrasound signal to the material to be treated or imaged; and a controller for electrically controlling the electroactive material actuator to implement resonant frequency tuning by change of layer thickness through actuation of the at least one electroactive material layer with adjustable thickness, to implement a shift of the frequency upon transmission of the ultrasound signal to the material to be treated or imaged.

This acoustic coupling component enables setting of an ultrasound impedance, so that the device can deliver improved acoustic performance, in that it can provide a shift or a tuning of the ultrasound frequency transmitted by actuation of the actuator. The shift or tuning may be performed in a dynamic way. This may for example hold for a resonance ultrasound frequency transmitted by the actuator.

The acoustic coupling component for example delivers a desired acoustic impedance, which can be set by design. A set acoustic impedance may be obtained by filling or lamination of the electroactive material, in particular, using sub-wavelength layers or particles. The actuation function enables shape control. In particular, the actuation of the device is used to control a frequency dependent behavior, by change of layer thickness through actuation. The ultrasound frequency may depend on an imaging mode or a treatment mode being used and the actuation of the component may then enable setting the component to be optimal for the particular mode being used.

The acoustic coupling component is designed to introduce deliberately caused reflections. Some frequencies are brought into resonance and hence increase the output pressure for that frequency. Other frequencies having a negative interference and hence have a lowered output pressure. In this way a narrow bandwidth response is created with higher output pressure. The frequencies are influenced by the thickness of the layers of the component and at least one of the layers, by way of it being an electroactive actuator, has adjustable thickness to therewith change and shift the ultrasound frequencies. In accordance with the invention, a shifted frequency is a resonance frequency. The actuator structure may be called a resonant structure.

Examples in accordance with another aspect of the invention provide an ultrasound device, comprising:

> a transducer arrangement; and
> an acoustic coupling component as defined above adapted to be positioned between the transducer arrangement and the material to be treated or imaged.

This device makes use of the tunable acoustic coupling component, so that the overall device can be tuned to deliver optimum performance as a function of the ultrasound frequency. This ultrasound frequency may depend on an imaging mode or a treatment mode being used.

For setting the acoustic impedance, the acoustic coupling component may comprise a layer structure of at least two different materials of different acoustic impedance. The total contribution to the overall thickness of each type of material controls the combined effective acoustic impedance.

In this structure, the layer thickness may be selected to be a small fraction of the wavelength of the ultrasound, for example less than 1/5 or 1/10, to reduce reflections in the structure.

This structure sets the acoustic impedance to a desired value. The sub-wavelength thicknesses of the layers in the structure are intended to avoid reflections.

By way of example, a first of the at least two materials of the structure comprises a PVDF relaxor electroactive polymer material and a second of the at least two different materials comprises a silicone dielectric electroactive polymer material. This material includes VDF units in the polymer backbone. There may be other units such as tirfluoroethylene as well. The PVDF material for example has an acoustic impedance of around 4 MRayls and the silicone material for example has an acoustic impedance of around 1 MRayls.

The acoustic coupling component comprises a resonance structure. This can serve to amplify an acoustic signal thereby improving the signal to noise for imaging applications and increasing the signal strength or reducing attenuation for tissue treatment applications.

For this purpose, the acoustic coupling component may have a single electroactive material layer (or a single type of multilayer structure which provides a general acoustic impedance), or it may comprise a layer arrangement of at least two different materials of different acoustic impedance (or two different types of multilayer structure). The layers of the "arrangement" (or multilayer structures) are thicker than the layers of the "structure" so as to form reflection interfaces.

In a first example, the acoustic coupling component comprises a layer arrangement of at least two different materials of different acoustic impedance. In this way, boundary reflections are established. The thickness of the actuated layers varies in dependence on the actuation, and this then influences the frequency characteristics of the layer arrangement in a predictable way.

In one implementation, a first of the at least two different materials is an electroactive material and has associated actuator electrodes, and a second of the at least two different materials is a passive layer. Thus, there may be a single type of actuated layer.

In another implementation, both first and second of the at least two different materials are electroactive materials each with associated actuator electrodes. Thus, there may be two or more different types of actuated layer. They may expand or contract together to reduce stresses in the layer arrangement, but they have different acoustic impedance.

The layer arrangement may comprise a top and bottom layer of first acoustic impedance and a middle layer of second, higher or lower acoustic impedance. This defines a three-layer arrangement. The middle layer may be actuated, and the top and bottom layers may be passive, or the top and bottom layers may be actuated and the middle layer may be passive, or they may all be actuated. Reflections arise at both surfaces of the middle layer and a resonant frequency is established. Resonance may also arise in the top and/or bottom layers.

In all examples above, the electroactive material composite may comprise an electroactive material layer having filler particles for setting the acoustic impedance.

When the transducer arrangement is adapted to generate ultrasound waves having a minimum wavelength in the tissue, the filler particles have a maximum linear dimension of less than 20% of said minimum wavelength. This prevents scattering of the ultrasound beam enabling control of the acoustic impedance.

The filler particles for example comprise ceramic particles.

Alternatively, the filler particles may comprise metal particles coated with a non-conductive coating. These may have a higher density and may therefore be needed in a lower concentration to deliver a desired shift in acoustic impedance.

There may be a segmented electrode arrangement associated with the electroactive material layer. The filler particles may have a non-uniform density across the area of the electroactive material layer as a result of the manufacturing process. This causes a non-uniform deformation profile to be established. The segmented electrode arrangement can be used to compensate for this, in a calibration process.

The acoustic coupling component using filler particles may comprise a layer arrangement of two different materials of different overall acoustic impedance (as explained above). One of the materials may comprise an electroactive material layer with the particles and the other may comprise the electroactive material layer without the particles.

By way of example, the electroactive material actuator typically comprises a relaxor PVDF electroactive polymer layer.

In one set of examples, the acoustic coupling component comprises an acoustically transmissive window over the transducer arrangement. The setting of the acoustic impedance is used to provide impedance matching between the window and the material, or to provide acoustic resonance.

In another set of examples, the acoustic coupling component is spaced from the transducer arrangement. This is of interest for a resonance component, which introduces a signal amplification between the transducer arrangement and the tissue.

Use of acoustic resonance means that around the resonance frequency the signal is amplified. At half and 1.5 times this frequency the amplitude is reduced, as the amplification is based on a pattern of constructive and destructive interference. The total pressure output remains the same if ignoring losses.

BRIEF DESCRIPTION OF THE DRAWINGS

Examples of the disclosure will now be described in detail with reference to the accompanying drawings, in which:

Figure 1 shows an electroactive material device which is not clamped to a carrier layer;
Figure 2 shows an electroactive material device which is designed so that the expansion arises only in one direction;
Figure 3 shows schematically an ultrasound device making use of tunable impedance matching;
Figure 4 shows an example of a layer structure for use in the ultrasound device of Figure 3;
Figure 5 shows a first example of a layer arrangement for use in the ultrasound device of Figure 3;
Figure 6 shows a second example of a layer arrangement for use in the ultrasound device of Figure 3;
Figure 7 shows how the acoustic coupling component may be remote from the transducer arrangement; and
Figure 8 shows an ultrasound system using the device.

It should be understood that the Figures are merely schematic and are not drawn to scale. It should also be understood that the same reference numerals are used throughout the Figures to indicate the same or similar parts.

DETAILED DESCRIPTION OF THE EMBODIMENTS

The disclosure provides an ultrasound device for treatment or imaging of material such as tissue, comprising a transducer arrangement and an acoustic coupling component positioned between the transducer arrangement and the material. The acoustic coupling component comprises an electroactive material actuator which uses a layered electroactive material structure composite. It is controlled to implement frequency tuning of the acoustic coupling component. In this way, the acoustic coupling can be optimized for different frequencies of operation. The disclosure also relates to the acoustic coupling component itself.

In particular, actuation of the electroactive material actuator causes a change in thickness of the layer, which changes the behavior of a resonant component using the layer.

The invention makes use of an actuator using an electroactive material (EAM). This is a class of materials within the field of electrically responsive materials. When implemented in an actuation device, subjecting an EAM to an electrical drive signal can make them change in size and/or shape. This effect can be used for actuation and sensing purposes.

There exist inorganic and organic EAMs.

A special kind of organic EAMs are electroactive polymers (EAPs). Electroactive polymers (EAP) are an emerging class of electrically responsive materials. EAPs, like EAMs can work as sensors or actuators, but can be more easily manufactured into various shapes allowing easy integration into a large variety of systems. Other advantages of EAPs include low power, small form factor, flexibility, noiseless operation, and accuracy, the possibility of high resolution, fast response times, and cyclic actuation. An EAP device can be used in any application in which a small amount of movement of a component or feature is desired, based on electric actuation. Similarly, the technology can be used for sensing small movements. The use of EAPs enables functions which were not possible before, or offers a big advantage over common sensor / actuator solutions, due to the combination of a relatively large deformation and force in a small volume or thin form factor, compared to common actuators. EAPs also give noiseless operation, accurate electronic control, fast response, and a large range of possible actuation frequencies, such as 0 - 20 kHz.

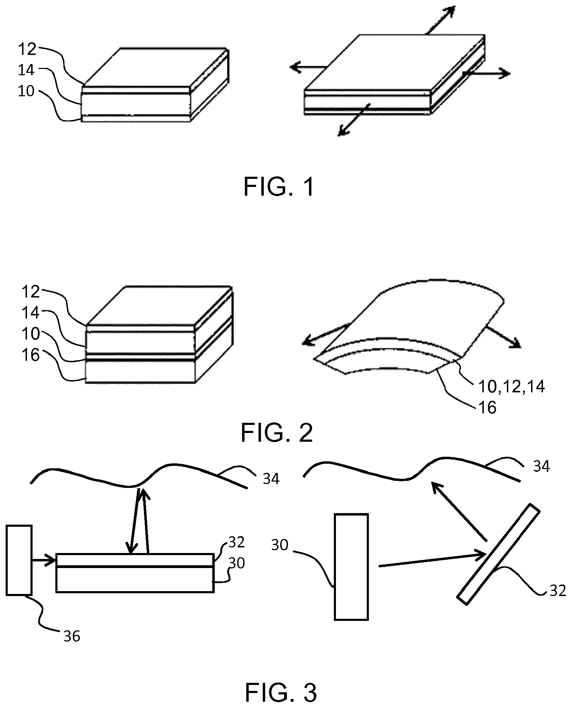

As an example of how an EAM device can be constructed and can operate, Figures 1 and 2 show two possible operating modes for an EAP device that comprises an electroactive polymer layer 14 sandwiched between electrodes 10, 12 on opposite sides of the electroactive polymer layer 14.

Figure 1 shows a device which is not clamped to a carrier layer. A voltage is used to cause the electroactive polymer layer to expand in all directions as shown.

Figure 2 shows a device which is designed so that the expansion arises only in one direction. To this end the structure of Figure 1 is clamped or attached to a carrier layer 16. A voltage is used to cause the electroactive polymer layer to curve or bow. The nature of this movement arises from the interaction between the active layer which expands when actuated, and the passive carrier layer which does not.

Figure 3 shows an ultrasound device for treatment or imaging of tissue, in two possible configurations. It comprises an ultrasound transducer arrangement 30 and an acoustic coupling component 32 positioned between the transducer arrangement 30 and the tissue 34 to be treated or imaged. The acoustic coupling component 32 comprises an electroactive material actuator, which in particular makes use of an electroactive material composite. It enables impedance matching and the actuation is then for beam steering or shaping or for resonant frequency tuning, in all cases to alter properties of the acoustic path between the transducer and the tissue.

The electroactive material composite uses at least two different materials. One is an electroactive material and the other is a further material for acoustic impedance setting. The setting may be achieved using a thin layer structure or by using filler particles. These options are discussed further below. At least one layer is actuatable, i.e. able to deliver a shape change in response to a drive signal.

A controller 36 is provided for controlling the electroactive material actuator thereby to implement the shape control of the acoustic coupling component 32. The shape control results in frequency tuning.

The left part of Figure 3 shows the acoustic coupling component 32 against an output window of the transducer arrangement 30. The right part of Figure 3 shows the acoustic coupling component 32 remote from the transducer arrangement, but in the path between the tissue 34 and the transducer arrangement 30.

This device enables tuning of the ultrasound coupling component 32, so that the device can be tuned to deliver optimum performance as a function of the ultrasound frequency. This ultrasound frequency may depend on an imaging mode or a treatment mode being used.

The acoustic coupling component presents a desired acoustic impedance. By combining sub layers of different types of electroactive material in a multilayer structure the impedance can be set to a desired value.

Once set, by the choice and dimensions of the layers, the overall structure can be actuated to alter the layer thicknesses. As explained below, this actuation is used to alter an ultrasound frequency which according to the invention is a resonance frequency.

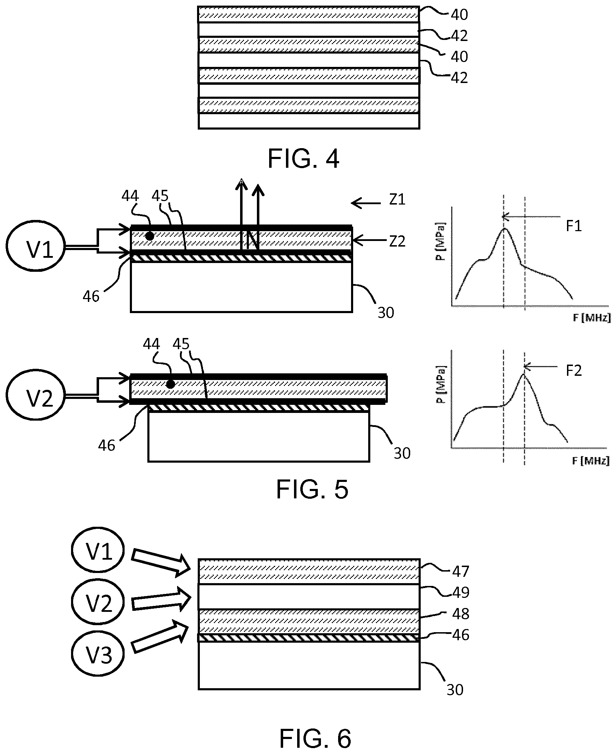

Figure 4 shows an electroactive multilayer structure comprising a first type 40 of electroactive material with acoustic impedance Z1 and a second type 42 of electroactive material with acoustic impedance Z2, arranged in an alternate stack. For example, one material type is a silicone (a dielectric electroactive material) and the other type is a relaxor ter-polymer such as poly(vinylidene fluoride-trifluoroethylene-chlorofluoroethylene) [P(VDF-TrFE-CFE)]).

If number and thickness of the (sub) layers is equal, the impedance is (Z1+Z2)/2. In this example, a silicone has an acoustic impedance of around Z1=1 MRayls and PVDF has an acoustic impedance of around Z2=4MRayls, giving a result of 2.5 MRayls.

By varying thicknesses or the number of layers, the impedance can be designed to be somewhere from Z1 to Z2.

In particular:

$Zavg = (d1Z1 + d2Z2)/(d1+d2)$ and d1 is the sum of thicknesses of layers with acoustic impedance Z1 and d2 is the sum of thicknesses of layers with acoustic impedance Z2.

This structure is intended only for fixed acoustic impedance setting.

It is described as a "layer structure", but the layers may also be in the form of a composite structure, for example using filler particles. The layer structure presents a general overall acoustic impedance, and does not provide significant internal acoustic reflections. It may therefore be considered to be equivalent to a single bulk material layer with a single effective acoustic impedance.

In order to prevent reflections at the interface between layers the thickness of these layers is preferably smaller than 1/5 of the wavelength, and more preferable less than 1/10. The difference in impedance of the overall multilayer structure with the surrounding determines the level of reflection.

As an example, in silicones the speed of sound is close to 1000 m/sec. At a frequency of 10 MHz the wavelength is 100 micrometers. The individual silicone layer should therefore be thinner than 10 microns. In the ter-polymer layer the speed of sound will be close to a factor of four higher. In that case the wavelength is also smaller with the same factor and these layers should then be thinner than 2.5 microns for use at 10 MHz.

Each sub layer may have its own electrode arrangement. The voltages applied to the different sub layers may need to be the same or different in order to obtain the same deformation of all layers. In particular, the different layers are likely to have a different deformation response to applied voltages.

The layer structure may have only one type of actuatable material, and the other material may be passive (by which is meant that it is not electrically controlled). This still enables tuning of the thickness but the maximum deformation level will be lower.

The material is for example acoustically matched to the acoustic impedance of the transducer array, i.e. has an acoustic impedance that approximately matches the acoustic impedance of the transducer array. For example, in the case of the transducer array comprising piezoelectric transducers, the electroactive material layer may have an acoustic impedance ranging from 1.3 - 3.0 MRayls, whereas in the case of the transducer array comprising CMUT elements, the layer may have an acoustic impedance ranging from 1.3 - 1.9 MRayls, which has the further advantage that the acoustic impedance is closely matched to that of body tissue, which typically has acoustic impedance of about 1.6 MRayls.

The "layer structure" of Figure 4 thus sets a desired impedance by having a combination of very thin layers. Actuation of the device does not change the acoustic impedance. The difference between the set impedance and the surrounding media determines level of reflection.

As will be described below, one use of the structure of Figure 4 is to create a resonant structure. The thickness of the layer in which reflections occur then determines a frequency of resonance.

The acoustic coupling component comprises a resonance structure. This resonance structure may have one or more layers with reflections at the layer boundaries. Each layer may be a single material layer, or it may be a "layer structure" of the type described above. Together, multiple layers may be considered to form a "layer arrangement". Thus, the term "layer structure" is used to denote a structure in which the layers simply aim to set the overall effective acoustic impedance, whereas the term "layer arrangement" is used to denote layer of greater thickness and the thickness is controlled to effect a resonance frequency control. Any one "layer" of the "layer arrangement" may in fact comprise a complete "layer structure" but it can be treated as a single layer with a single effective acoustic impedance.

The electroactive material layer is selected with a different impedance to its surroundings. It has a larger thickness so that reflections are not suppressed. These surroundings may be other materials which are part of the device or may be tissue, skin or blood, etc. The impedance mismatches create reflections.

Reflections lead to a resonance with a frequency depending on the layer thickness. Resonance occurs at 2d = λ where d is the thickness of the layer and λ the wavelength.

As f = v/λ, (f is the frequency and v is the sound velocity) the resonance frequency is f = v/2d [in units of 1/s].

The electroactive material actuator for example has electrodes in the form of conductive thin films on either side. Upon actuation, the thickness of the material film decreases, shifting the acoustic resonance to a higher frequency. By controlling the applied voltage, a resonance frequency between the non-actuated situation and the maximum actuated situation can be selected. Resonance frequency sweeps are also possible.

The electrodes are acoustically transparent as they are very thin for instance sub-micron metal layers. It is also an option to apply (thicker) electrodes with impedance close to the electroactive material for example by using conducting polymers.

The level of reflection at the interfaces between the electroactive material layer and its surrounding can be controlled by selecting a desired impedance of the electroactive material layer.

One option is to form the layer from sub-wavelength sub-layers as explained above to create a layer structure which sets the overall acoustic impedance. Thus, the resonance structure comprises one or more layers, or layer structures, of the type of Figure 4. Another option for setting the acoustic impedance is to provide filler particles. This is discussed further below.

The level of resonance is determined by the reflection coefficient R which is directly related to the impedance difference between the electroactive material layer (Z2) and its surroundings (Z1):

$$R = \left| \frac{Z1 - Z2}{Z1 + Z2} \right|$$

Figure 5 shows a device having a single electroactive material layer 44 of acoustic impedance Z2 (or a layer structure of effective acoustic impedance Z2) surrounded by electrodes 45. The layer 44 may be a single layer or a layer structure of the type explained above. This layer 44 is coupled to the ultrasound transducer 30 by a viscous coupling layer 46, allowing relative sliding between the two layers on each side. The acoustic impedance beyond the structure has acoustic impedance Z1.

The top image shows a non-actuated state with voltage V1 (e.g. V1=0) and a first resonance frequency F1. The bottom image shows an actuated state with voltage V2 and a second resonance frequency F2. The actuation results in a higher resonance frequency.

Figure 6 shows a multilayer resonance "layer arrangement".

There is a top material layer 47, a bottom material layer 48 and a middle layer 49. The middle layer 49 is of a first material and is thus sandwiched between two layers 47,48 of a second material with higher impedance, and there is again a viscous coupling layer 46 which couples the electroactive material stack to the ultrasound transducer 30. Three different drive voltages are shown as VI, V2 and V3. They may be the same or different.

Reflections occur at both interfaces of the middle layer 49. The thickness of the low impedance layer 49 determines a resonant frequency according to 2d = $\lambda$, $f_{res}$ = v/2d as explained above.

Also in the bottom higher impedance layer 48 reflections occur leading to a resonance with frequency determined by the thickness and sound velocity.

Whether there will also be a reflection in the top layer 47 depends on the impedance mismatch with the surrounding material. The different layers may be set in such a way that the resonance frequencies are equal i.e. v/2d is constant.

The three layers can be actuated in parallel leading to a very homogeneous deformation of the sandwiched layer 49 and therefore a very sharp resonance, and the layer 49 maintains its rectangular shape. The middle layer in this example has the lower impedance Z2 and the top and bottom layers have higher impedance Z1.

As explained above, one or more of the layers of Figure 6 may be layer structures of the type explained with reference to Figure 4.

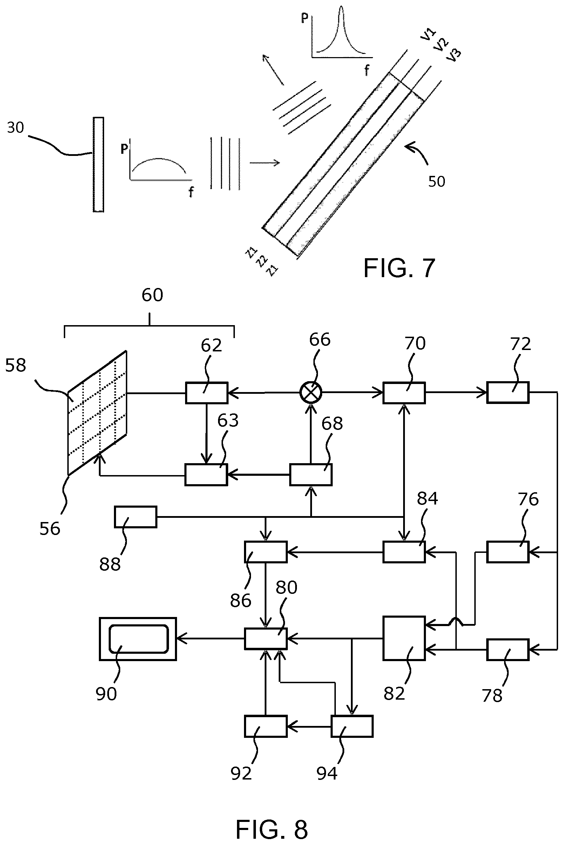

Figure 7 shows the resonant layer arrangement structure 50 of Figure 6 separate from the ultrasound transducer 30. In principle all examples may be physically attached to or detached from the transducer.

The remote resonant structure 50 converts an incident acoustic wave with a relatively flat pressure-frequency response into a sharper pressure-frequency response at the resonant frequency, as shown in Figure 7.

The impedance Z1 of the top and bottom layers may in this example be set to the impedance of the environment and therefore not resonant. The middle layer has an impedance Z2 which is lower or higher, and therefore this layer will cause resonance at certain frequencies.

The voltages applied to all three layers may be selected to achieve homogeneous deformation of the middle layer by ensuring the same deformation level in all three layers. For example, electroactive material layers with one specific impedance can show differences in actuation characteristics compared to similar layers set to another impedance. Different voltage drive levels (V1, V2, V3) can be used to compensate for this.

However, it is also possible to actuate only the middle layer (and have passive layers outside), or actuate only the outer layers (and have a passive middle layer), with the actuation causing the non-actuated layers to deform based on mechanical or chemical coupling between the layers. There may be only one electroactive material layer and one passive layer. Multiple such pairs of layers may be stacked to obtain a sharper resonance (lower bandwidth and higher pressure at the resonance frequency).

Another option for setting/tuning the acoustic impedance (i.e. instead of the multilayer structure of Figure 4) is to use filler particles within a matrix formed by the electroactive material layer. The filler comprises particles having a selected material and particle density to achieve a desired change in acoustic impedance. The filler aims to increase the acoustic impedance of the electroactive material layer without significantly reducing its actuation response.

The filler particles then function as the further material for acoustic impedance setting. The resulting structure may be considered to be a composite structure.

Any suitable non-conductive particles or mixture of non-conductive particles may be used for this purpose. By way of non-limiting example, the non-conductive particles may be ceramic particles, e.g. transition metal oxides, nitrides, carbide particles, or high-density metal oxide such as tungsten oxides, bismuth oxides. Alternatively, the filler particles may comprise metal particles such as tungsten coated with a non-conductive coating. These may have a higher density and may therefore be needed in a lower concentration to deliver a desired shift in acoustic impedance. Finally, a low concentration of non-coated conducting particles may be used.

The particles increase the weight and therefore the acoustic impedance (Z=p.v [MRayls]) so that reflections with the surrounding materials are tuned as explained above.

The tuning of the resonance frequency takes place using the same mechanism as explained above.

Fillers particles that have a high density are preferred as lower concentrations are required for the same acoustic impedance increase. The impedance of the composite layer is the density of the composite times the speed of sound in the composite.

The use of an electroactive material layer having an impedance set using a filler may be applied to all examples above of resonance structure. For example, such a composite may form:

the electroactive material layer 44 of Figure 5;
the electroactive material layers 60 and/or 62 and/or 64 of Figure 6.

For the three-layer arrangement of Figure 6, the outer layers may have a set impedance using filler particles. For example, each of these two layers may have an impedance set to the medium on that side of the component. The thickness of the middle layer is then controlled to influence the resonance frequency.

Alternatively, the middle layer may have a set impedance using filler particles.

As in the examples above in a stacked structure some or all layers may be actuated. Thus, some layers may be passive layers.

The transducer arrangement is for example adapted to generate ultrasound waves having a minimum wavelength in the tissue. The filler particles have a maximum linear dimension of less than 20% of said minimum wavelength, for example less than 10%. For example, ultrasound waves in the 7-12 MHz range correspond to a wavelength in the body of about 0.1-0.2 mm. The maximum particle size of the particles in the electroactive material layer is then preferably less than 0.02mm or even 0.01mm. This prevents scattering of the ultrasound beam enabling control of the acoustic impedance.

There may be a segmented electrode arrangement associated with the electroactive material layer having filler particles. The filler particles may have a non-uniform density across the area of the electroactive material layer as a result of the manufacturing process. This causes a non-uniform deformation profile to be established. The segmented electrode arrangement can be used to compensate for this, in a calibration process. A different potential is then applied to each segment until the thickness of the EAP is as uniform as possible. A calibration step may for example use variable resistors or capacitors in series which then remain fixed during use. The spatial separation of the different electrode areas is for example smaller or comparable to the thickness of the layer.

The disclosure relates to the use of a structure for transmission of ultrasound signals and capable of changing the frequency such as a resonance frequency. However, the same concept of impedance setting of an actuatable layer may be used in other applications. For example, by providing a shape change, for example an angle change of at least one interface, a beam steering or acoustic lens focusing or defocusing function may be implemented. A set impedance for such actuatable components is of general interest. The deflection angle at the interface is determined by the speed of sound difference between materials at the interface. By adding filler particles, the velocity of sound may be changed somewhat, but by combining sub-wavelength layers as described above, a larger effect on the sound velocity can be obtained.

The ability to set the acoustic impedance by using a composite structure is thus of interest for any actuatable component to be used in an ultrasound environment.

In all examples, the electroactive material actuator is typically based on an electroactive polymer material, although the invention can in fact be used for devices based on other kinds of EAM material. Such other EAM materials are known in the art and the person skilled in the art will know where to find them and how to apply them. A number of options will be described herein below.

A common sub-division of EAM devices is into field-driven and current or charge (ion) driven EAMs. Field-driven EAMs are actuated by an electric field through direct electromechanical coupling, while the actuation mechanism for current or charge driven EAMs involves the diffusion of ions. The latter mechanism is more often found in the corresponding organic EAMs such as EAPs. While field driven EAMs generally are driven with voltage signals and require corresponding voltage drivers/controllers, current driven EAMs generally are driven with current or charge signals sometimes requiring current drivers. Both classes of materials have multiple family members, each having their own advantages and disadvantages.

Field driven EAMs can be organic or inorganic materials and if organic can be single molecule, oligomeric or polymeric. For the current invention they are preferably organic and then also oligomeric or even polymeric. The organic materials and especially polymers are an emerging class of materials of growing interest as they combine the actuation properties with material properties such as light weight, cheap manufacture and easy processing.

The field driven EAMs and thus also EAPs are generally piezoelectric and possibly ferroelectric and thus comprise a spontaneous permanent polarization (dipole moment). Alternatively, they are electrostrictive and thus comprise only a polarization (dipole moment) when driven, but not when not driven. Alternatively, they are dielectric relaxor materials. Such polymers include, but are not limited to, the sub-classes: piezoelectric polymers, ferroelectric polymers, electrostrictive polymers, relaxor ferroelectric polymers (such as PVDF based relaxor polymers or polyurethanes), dielectric elastomers, liquid crystal elastomers. Other examples include electrostrictive graft polymers, electrostrictive paper, electrets, electroviscoelastic elastomers and liquid crystal elastomers.

The lack of a spontaneous polarization means that electrostrictive polymers display little or no hysteretic loss even at very high frequencies of operation. The advantages are however gained at the expense of temperature stability. Relaxors operate best in situations where the temperature can be stabilized to within approximately 10 °C. This may seem extremely limiting at first glance, but given that electrostrictors excel at high frequencies and very low driving fields, then the applications tend to be in specialized micro actuators. Temperature stabilization of such small devices is relatively simple and often presents only a minor problem in the overall design and development process.

Relaxor ferroelectric materials can have an electrostrictive constant that is high enough for good practical use, i.e. advantageous for simultaneous sensing and actuation functions. Relaxor ferroelectric materials are non-ferroelectric when zero driving field (i.e. voltage) is applied to them, but become ferroelectric during driving. Hence there is no electromechanical coupling present in the material at non-driving. The electromechanical coupling becomes non-zero when a drive signal is applied and can be measured through applying the small amplitude high frequency signal on top of the drive signal. Relaxor ferroelectric materials, moreover, benefit from a unique combination of high electromechanical coupling at non-zero drive signal and good actuation characteristics.

The most commonly used examples of inorganic relaxor ferroelectric materials are: lead magnesium niobate (PMN), lead magnesium niobate-lead titanate (PMN-PT) and lead lanthanum zirconate titanate (PLZT). But others are known in the art.

PVDF based relaxor ferroelectric based polymers show spontaneous electric polarization and they can be pre-strained for improved performance in the strained direction. They can be any one chosen from the group of materials herein below.

Polyvinylidene fluoride (PVDF), Polyvinylidene fluoride - trifluoroethylene (PVDF-TrFE), Polyvinylidene fluoride - trifluoroethylene - chlorofluoroethylene (PVDF-TrFE-CFE), Polyvinylidene fluoride - trifluoroethylene - chlorotrifluoroethylene) (PVDF-TrFE-CTFE), Polyvinylidene fluoride- hexafluoropropylene (PVDF - HFP), polyurethanes or blends thereof.

The sub-class dielectric elastomers includes, but is not limited to acrylates, polyurethanes, silicones.

Examples of ionic-driven EAPs are conjugated polymers, carbon nanotube (CNT) polymer composites and Ionic Polymer Metal Composites (IPMC).

The sub-class conjugated polymers includes, but is not limited to:

polypyrrole, poly-3,4-ethylenedioxythiophene, poly(p-phenylene sulfide), polyanilines.

The materials above can be implanted as pure materials or as materials suspended in matrix materials. Matrix materials can comprise polymers.

To any actuation structure comprising EAM material, additional passive layers may be provided for influencing the behavior of the EAM layer in response to an applied drive signal.

The actuation arrangement or structure of an EAM device can have one or more electrodes for providing the control signal or drive signal to at least a part of the electroactive material. Preferably the arrangement comprises two electrodes. The EAM layer may be sandwiched between two or more electrodes. This sandwiching is needed for an actuator arrangement that comprises an elastomeric dielectric material, as its actuation is among others due to compressive force exerted by the electrodes attracting each other due to a drive signal. The two or more electrodes can also be embedded in the elastomeric dielectric material. Electrodes can be patterned or not.

It is also possible to provide an electrode layer on one side only for example using interdigitated comb electrodes.

A substrate can be part of the actuation arrangement. It can be attached to the ensemble of EAP and electrodes between the electrodes or to one of the electrodes on the outside.

The electrodes may be stretchable so that they follow the deformation of the EAM material layer. This is especially advantageous for EAP materials. Materials suitable for the electrodes are also known, and may for example be selected from the group consisting of thin metal films, such as gold, copper, or aluminum or organic conductors such as carbon black, carbon nanotubes, graphene, poly-aniline (PANI), poly(3,4-ethylenedioxythiophene) (PEDOT), e.g. poly(3,4-ethylenedioxythiophene) poly(styrenesulfonate) (PEDOT:PSS). Metalized polyester films may also be used, such as metalized polyethylene terephthalate (PET), for example using an aluminum coating.

The materials for the different layers will be selected for example taking account of the elastic moduli (Young's moduli) of the different layers.

Additional layers to those discussed above may be used to adapt the electrical or mechanical behavior of the device, such as additional polymer layers.

The device described above can be applied in a wide range of medical ultrasound applications for example (but not limited to) on body, in the esophagus (TEE, transesophageal echocardiogram) wearable ultrasound, large area ultrasound. Different transducer types may be used such as PZT, single crystal, CMUT.

Some examples above make use of composite materials which combine an electroactive material (in particular a polymer) and other particles (which have been termed generally as a "filler") for changing the acoustic impedance.

The way such composite materials can be manufactured will now be discussed as well as the effects on the physical and electrical properties of the electroactive material.

The example of dielectric elastomer electroactive materials will first be presented. These are sandwiched between two electrodes to create dielectric electroactive polymer actuators. Silicone rubbers are the main applied elastomer group. The deformation is the result of attractive forces between the positively and negatively charged electrodes.

Compounding of particles in silicones is widely used on an industrial scale. As an example ultrasound transducer lenses are made of silicone (PDMS, Polydimethylsiloxane) filled with iron and silicon oxide particles to increase acoustic impedance and wear resistance. PDMS (silicone) compounds containing rutile (TiO2) are widely used to increase the refractive index or to create white reflecting materials.

With respect to the performance of a dielectric electroactive polymer, compounding with non-conducting hard particles such as ceramics has two main significant effects. First, the stiffness of the material increases requiring larger forces to obtain the same strain levels. Another effect is that the dielectric constant of the composite changes (in general that of the filler will be higher than that of silicones, which is close to 3). Whether the strain effect depending on voltage is positive or negative depends on the dielectric constant of the particles and on particle size as more small particles have a larger effect on stiffness.

This is discussed in S. Somiya, "Handbook of Advanced Ceramics: Materials, Applications, Processing, and Properties," in Nonlinear Dielectricity of MLCCs, Waltham, Academic Press, 2013, p. 415. By way of example, adding particles increases the dielectric constant but also increases the stiffness.

Thus, compounding fillers into elastomers to influence the properties of a dielectric electroactive polymer is known. Adding high dielectric constant particles to increase the dielectric constant of the elastomer and therefore potentially the effectivity, has been widely investigated.

Silicone elastomers are in general prepared by mixing two components. One of them contains a Pt or peroxide curing catalyst. The different components can be mixed in a high speed mixer. In the same process, the filler can be added or the filler may already be premixed in one or both components. The filler material is in general applied in a solvent which evaporates during processing. After or during mixing in a high speed mixer in general vacuum is applied to remove air (and or solvents) inclusions. After this the mixture can be casted and cured. Curing temperature and time depends on the polymer grade but is typically around 80 °C for 10 minutes. Most particles are compatible with silicones as long as they do not inactivate the catalyst (for instance sulfur containing materials). Peroxide curing silicones are less sensitive.

Silicones can be injection molded (liquid silicone rubbers, LSR). The two components are injected on a screw, after passing a (static) mixer, of the LSR injection molding machine. The filler particles may be premixed in one or both components. The material is transported by a cold screw and injected into a hot mold where it cures fast depending on temperature. As the LSR has very low viscosity very thin sections can be realized. Typical curing temperatures are close to 180 oC and times around 30 seconds to one minute.

Besides casting and injection molding a number of other shaping technologies are available to produce silicon rubber compound components also in the form of thin films. Examples are extrusion (foils and profiles), rolling of foils, lamination and rolling of multilayers, doctor blade film casting, spin coating and screen printing.

The combining of different material types can be performed locally at the point of manufacture, for example by using multi shot injection molding (2 shot or overmolding), silicone dispensing and over casting or silicone additive manufacturing (i.e. 3D printing).

The example of piezoelectric polymer composites will next be presented.

Piezo electric polymer composites containing a compound of PVDF (a matrix polymer) and ceramic particles such as PZT have been investigated. Manufacturing technologies like solvent casting and spin coating are suitable. Also, cold and hot pressing techniques are suitable. After dissolving the PVDF, evaporation of solvent until a viscous mix is obtained and mixing in the filler particles may then be performed. PVDF polymer based composites with a well dispersed grain size distribution and intact polymer matrix may be realized.

The example of relaxor electrostrictive polymer actuators will next be presented.

These are a class of semicrystalline terpolymers that can deliver a relatively high force with medium strain. Therefore, these actuators have a wide range of potential applications. Relaxor electrostrictive polymers have been developed from "normal" PVDF polymers by employing proper defect modifications. They contain: vinylidene fluoride (VDF), trifluoroethylene (TrFE), and 1, 1-chlorofluoroethylene (CFE) or Chlorotrifluoro ethylene (CTFE).

Addition of defects in the form of chemical monomers, like 1, 1-chlorofluoroethylene (CFE) which are copolymerised with the VDF-TrFE, eliminate the normal ferroelectric phase, leading to a relaxor ferroelectric with electromechanical strain greater than 7% and an elastic energy density of 0.7 $J/cm^3$ at 150 MV/m. Furthermore, it has been described that by introducing defects via high electron irradiation of the P(VDF-TrFE) copolymers, the copolymer can also be converted from a "normal" ferroelectric P(VDFTrFE) into a ferroelectric relaxor.

The materials may be formed by polymer synthesis as described in F. Carpi and et. al., "Dielectric Elastomers as Electromechanical Transducers: Fundamentals, Materials, Devices, Models and Applications of an Emerging Electroactive Polymer Technology," Oxford, Elsevier, 2011, p. 53. This discloses a combination of a suspension polymerization process and an oxygen-activated initiator. This films can be formed by pouring the solution on a glass substrate and then evaporating the solvent.

The desired filler can be added to the solvent before film casting. After casting, the composite can then be annealed to remove the solvent and increase crystallinity. The crystallization rate can reduce depending on filler concentration and particle size distribution. Stretching will align molecule chains and will become more difficult as particles can pin molecular chains. The dielectric constant will increase for most additives which reduces the required actuation voltage to reach a certain strain. The material stiffness will increase reducing strain.

The manufacturing process thus involves forming a polymer solution, adding particles, mixing, followed by casting (e.g. tape casting) potentially combined with lamination. Alternatives are spin coating, pressing etc.

Local variations in concentration can be realized using dispensing and or 3D solvent printing. Layer thicknesses between 10 to 20$\mu$m are for example possible with 3D printing processes.

In all examples, the addition of the filler generally has an effect on the breakdown voltage. The maximum strain that can be reached with an electroactive polymer is determined by the maximum voltage that can be applied, which is the breakdown voltage (or dielectric strength).

The breakdown voltage of polymers is related to the dissociation of polymer molecules under an applied external field. The addition of filler particles in a polymer matrix can have a significant influence on the breakdown voltage. Especially larger particles can locally increase fields. Therefore, compounding polymers with particles in the sub-micron range has a lower negative effect on voltage breakdown. Furthermore, the polymer - filler interface structure can strongly influence voltage breakdown.

Agglomeration of particles is another effect that reduces breakdown voltage. However, by modifying particle surfaces, preventing agglomeration and improving the interface structure, the negative effect of voltage breakdown levels can be reduced. However, the filled polymers will obtain a lower breakdown strength then unfilled polymers, leading to lower actuation strain.

In conclusion, for dielectric electroactive polymers, compounding with particles can be achieved using a wide range of industrial compounding and shaping technologies. In order to keep the effect on stiffness and therefore stroke reduction for an actuator limited, smaller concentrations are preferred. For a given volume concentration, not too small particles are also preferred to keep the effect on stiffness limited. A soft base polymer can be selected to compensate for the rise in stiffness. Increased dielectric constant can enable actuation at reduced voltages. In order to maintain the dielectric strength, particle size and concentration should be limited and measures can be taken to improve the polymer - filler interface as well as particle dispersion. Local concentration variations can be printed.

For relaxor type electro active polymers compounding with particles is also possible. Similar trends with respect to the influence of particle concentration and size, on stiffness and dielectric strength are comparable to the effects described above. Particles can be added after polymerization. Dissolved polymers can be shaped using various technologies such as tape casting and spin coating. Also local concentration variations are possible.

The ultrasound device of the disclosure may be an ultrasound probe or the like for use in an ultrasound imaging system or an ultrasound therapy system. The ultrasound probe may form part of a catheter for invasive imaging or treatment, may form part of a hand-held device for non-invasive imaging or treatment or may form part of a wearable device, e.g. for prolonged treatment of particular area of the body of a patient.

The ultrasound device may form part of an ultrasound system such as an ultrasonic diagnostic imaging system or an ultrasonic therapy system.

An example embodiment of an ultrasonic diagnostic imaging system is schematically depicted in block diagram form in Figure 8.

A transducer array 56 comprising the ultrasound transducer tiles 58 is provided in an ultrasound device 60 in the form of a probe for transmitting ultrasonic waves and receiving echo information. The transducer array 56 may be a one- or a two-dimensional array of transducer elements, e.g. tiles 58, capable of scanning in a 2D plane or in three dimensions for 3D imaging.

The transducer array 56 is coupled to a microbeam former 62 in the probe 60 which controls transmission and reception of signals by the array cells, e.g. CMUT cells. Microbeam formers are capable of at least partial beam forming of the signals received by groups or "patches" of transducer elements for instance as described in US patents US 5,997,479 (Savord et al.), US 6,013,032 (Savord), and US 6,623,432 (Powers et al.) The microbeam former 62 is coupled by the probe cable, e.g. coaxial wire, to a transmit/receive (T/R) switch 66 which switches between transmission and reception modes and protects the main beam former 70 from high energy transmit signals when a microbeam former is not present or used and the transducer array 56 is operated directly by the main system beam former 70. The transmission of ultrasonic beams from the transducer array 56 under control of the microbeam former 62 is directed by a transducer controller 68 coupled to the microbeam former by the T/R switch 66 and the main system beam former 70, which receives input from the user's operation of the user interface or control panel 88. One of the functions controlled by the transducer controller 68 is the direction in which beams are steered and focused. Beams may be steered straight ahead from (orthogonal to) the transducer array 56, or at different angles for a wider field of view. The transducer controller 68 is coupled to control a voltage source 63 for the transducer array 56. For instance, the voltage source 63 sets DC and AC bias voltage(s) that are applied to the CMUT cells 58 of a CMUT array 56, e.g. to drive the CMUT cells into a collapse mode.

The partially beam-formed signals produced by the microbeam former 62 are forwarded to the main beam former 70 where partially beam-formed signals from individual patches of transducer elements are combined into a fully beam-formed signal. For example, the main beam former 70 may have 128 channels, each of which receives a partially beam-formed signal from a patch of dozens or hundreds of transducer cells, e.g. from tiles 58. In this way the signals received by thousands of transducer elements of a transducer array 56 can contribute efficiently to a single beam-formed signal.

The beam-formed signals are coupled to a signal processor 72. The signal processor 72 can process the received echo signals in various ways, such as bandpass filtering, decimation, I and Q component separation, and harmonic signal separation which acts to separate linear and nonlinear signals so as to enable the identification of nonlinear (higher harmonics of the fundamental frequency) echo signals returned from tissue and microbubbles.

The signal processor 72 optionally may perform additional signal enhancement such as speckle reduction, signal compounding, and noise elimination. The bandpass filter in the signal processor 72 may be a tracking filter, with its pass band sliding from a higher frequency band to a lower frequency band as echo signals are received from increasing depths, thereby rejecting the noise at higher frequencies from greater depths where these frequencies are devoid of anatomical information.

The processed signals are coupled to a B-mode processor 76 and optionally to a Doppler processor 78. The B-mode processor 76 employs detection of an amplitude of the received ultrasound signal for the imaging of structures in the body such as the tissue of organs and vessels in the body. B-mode images of structure of the body may be formed in either the harmonic image mode or the fundamental image mode or a combination of both for instance as described in US Patents US 6,283,919 (Roundhill et al.) and US 6,458,083 (Jago et al.)

The Doppler processor 78, if present, processes temporally distinct signals from tissue movement and blood flow for the detection of the motion of substances, such as the flow of blood cells in the image field. The Doppler processor typically includes a wall filter with parameters which may be set to pass and/or reject echoes returned from selected types of materials in the body. For instance, the wall filter can be set to have a pass band characteristic which passes signal of relatively low amplitude from higher velocity materials while rejecting relatively strong signals from lower or zero velocity material.

This pass band characteristic will pass signals from flowing blood while rejecting signals from nearby stationary or slowing moving objects such as the wall of the heart. An inverse characteristic would pass signals from moving tissue of the heart while rejecting blood flow signals for what is referred to as tissue Doppler imaging, detecting and depicting the motion of tissue. The Doppler processor receives and processes a sequence of temporally discrete echo signals from different points in an image field, the sequence of echoes from a particular point referred to as an ensemble. An ensemble of echoes received in rapid succession over a relatively short interval can be used to estimate the Doppler shift frequency of flowing blood, with the correspondence of the Doppler frequency to velocity indicating the blood flow velocity. An ensemble of echoes received over a longer period of time is used to estimate the velocity of slower flowing blood or slowly moving tissue. The structural and motion signals produced by the B-mode (and Doppler) processor(s) are coupled to a scan converter 82 and a multiplanar reformatter 94. The scan converter 82 arranges the echo signals in the spatial relationship from which they were received in a desired image format. For instance, the scan converter may arrange the echo signal into a two dimensional (2D) sector-shaped format, or a pyramidal three dimensional (3D) image.

The scan converter can overlay a B-mode structural image with colors corresponding to motion at points in the image field with their Doppler-estimated velocities to produce a color Doppler image which depicts the motion of tissue and blood flow in the image field. The multiplanar reformatter 94 will convert echoes which are received from points in a common plane in a volumetric region of the body into an ultrasonic image of that plane, for instance as described in US Patent US 6,443,896 (Detmer). A volume renderer 92 converts the echo signals of a 3D data set into a projected 3D image as viewed from a given reference point as described in US Pat. 6,530,885 (Entrekin et al.)

The 2D or 3D images are coupled from the scan converter 82, multiplanar reformatter 94, and volume renderer 92 to an image processor 80 for further enhancement, buffering and temporary storage for display on an image display 90. In addition to being used for imaging, the blood flow values produced by the Doppler processor 78 and tissue structure information produced by the B-mode processor 76 are coupled to a quantification processor 84. The quantification processor produces measures of different flow conditions such as the volume rate of blood flow as well as structural measurements such as the sizes of organs and gestational age. The quantification processor may receive input from the user control panel 88, such as the point in the anatomy of an image where a measurement is to be made.

Output data from the quantification processor is coupled to a graphics processor 86 for the reproduction of measurement graphics and values with the image on the display 90. The graphics processor 86 can also generate graphic overlays for display with the ultrasound images. These graphic overlays can contain standard identifying information such as patient name, date and time of the image, imaging parameters, and the like. For these purposes the graphics processor receives input from the user interface 88, such as patient name.

The user interface is also coupled to the transmit controller 68 to control the generation of ultrasound signals from the transducer array 56 and hence the images produced by the transducer array and the ultrasound system. The user interface is also coupled to the multiplanar reformatter 94 for selection and control of the planes of multiple multiplanar reformatted (MPR) images which may be used to perform quantified measures in the image field of the MPR images.

As will be understood by the skilled person, the above embodiment of an ultrasonic diagnostic imaging system is intended to give a non-limiting example of such an ultrasonic diagnostic imaging system. The skilled person will immediately realize that several variations in the architecture of the ultrasonic diagnostic imaging system are feasible without departing from the teachings of the present disclosure. For instance, as also indicated in the above embodiment, the microbeam former 62 and/or the Doppler processor 78 may be omitted, the ultrasound probe 60 may not have 3D imaging capabilities and so on. Other variations will be apparent to the skilled person.

Moreover, in case of an ultrasonic therapy system, there obviously is no need for the system to be able to receive and process pulse echoes, such that it will be immediately apparent to the skilled person that the above embodiment of an ultrasonic diagnostic imaging system may be adapted to form an ultrasonic therapy system by omission of those system components that are required for the reception of processing of such pulse echoes.

The invention provides a tunable resonance function. Tunable resonance has significant benefits in a number of applications. In high intensity focused ultrasound treatment (like prostate cancer ablation) the resonance frequency can be adapted depending on the optimum absorption of the diseased tissue. The resonance frequency can also be adapted in situ for alternating imaging and treatment at the optimum frequency.

In ultrasound cell lysis the ultrasound output pressure may be controlled as a function of frequency to be tuned to the ultrasound absorption characteristic (cell resonance) of the specific cells for disintegration.

In imaging a frequency dependent output pressure can be adapted depending on the applied imaging mode, desired resolution or penetration depth.

The examples above are based on treatment or imaging of living tissue such as mammalian tissue, e.g. human tissue. The device may be used for treatment or imaging of non-living tissue and similarly the invention may be applied to apparatus for imaging non-biological material.

Ultrasound is for example used for material inspection. A lower frequency may be used for higher penetration depth whereas a higher frequency may be used for improved resolution. The resonance control described above is thus clearly of interest for such applications.

It should be noted that the above-mentioned embodiments illustrate rather than limit the invention, and that those skilled in the art will be able to design many alternative embodiments without departing from the scope of the appended claims.

## Claims

1. An acoustic coupling component (32) for positioning between an ultrasound transducer arrangement (30) and material (34) to be treated or imaged, the acoustic coupling component (32) comprising:

   an electroactive material actuator comprising a layered electroactive material resonance structure of at least one electroactive material layer with adjustable thickness, and a further material layer of different acoustic impedance, wherein the electroactive material actuator is adapted to receive an ultrasound signal having a frequency from the ultrasound transducer arrangement (30), and to transmit the ultrasound signal to the material (34) to be treated or imaged; and
   a controller (36) for electrically controlling the electroactive material actuator to implement resonant frequency tuning by change of layer thickness through actuation of the at least one electroactive material layer with adjustable thickness, to implement a shift of the resonance frequency upon transmission of the ultrasound signal to the material (34) to be treated or imaged.

2. An acoustic coupling component (32) as claimed in claim 1, wherein the layered electroactive material resonance structure has at least two different materials (40, 42) of different acoustic impedance.

3. An acoustic coupling component (32) as claimed in claim 2, wherein a first (40) of the materials of the layered electroactive material resonance structure comprises a PVDF electroactive polymer material, and a second (42) of the materials of the layered electroactive material resonance structure comprises a silicone dielectric electroactive polymer material.

4. An acoustic coupling component (32) as claimed in any preceding claim, wherein:

   the electroactive material has associated actuator electrodes and the further material layer is a passive layer, or
   both the electroactive material layer and the further material layer are electroactive materials each with associated actuator electrodes.

5. An acoustic coupling component (32) as claimed in claim 4, wherein the layered electroactive material resonance structure comprises a top (47) and bottom (48) layer of first acoustic impedance, and a middle layer (49) of second, lower acoustic impedance.

6. An acoustic coupling component (32) as claimed in any preceding claim, wherein the electroactive material layer and/or the further material layer comprise filler particles for tuning the acoustic impedance.

7. An acoustic coupling component (32) as claimed in claim 6, wherein the filler particles comprise ceramic particles or metal particles coated with a non-conductive coating.

8. An acoustic coupling component (32) as claimed in claim 6 or 7, comprising a segmented electrode arrangement associated with the electroactive material layer actuator.

9. An acoustic coupling component (32) as claimed in any one of claims 6 to 8, wherein the electroactive material actuator comprises an electroactive material layer with the particles, and an electroactive material layer without the particles.

**10.** An acoustic coupling component (32) as claimed in any preceding claim, wherein the electroactive material layer comprises a PVDF electroactive polymer layer.

**11.** An ultrasound device, comprising:

a transducer arrangement (30); and
an acoustic coupling component (32) as claimed in any preceding claim, adapted to be positioned between the transducer arrangement and the material to be treated or imaged.

**12.** An ultrasound device as claimed in claim 11, wherein the acoustic coupling component (32) is an acoustically transmissive window over the transducer arrangement (30).

**13.** An ultrasound device as claimed in claim 12, wherein the acoustic coupling component (32) is spaced from the transducer arrangement (30).

**14.** An ultrasound device as claimed in claim 11, 12 or 13, wherein the transducer arrangement (30) is adapted to generate ultrasound waves having a minimum wavelength in the material, wherein the electroactive material layer comprises filler particles having a maximum linear dimension of less than 20% of said minimum wavelength.

**Patentansprüche**

**1.** Akustische Kopplungskomponente (32) zum Positionieren zwischen einer Ultraschallwandleranordnung (30) und einem zu behandelnden oder abzubildenden Material (34), wobei die akustische Kopplungskomponente (32) umfasst:

einen elektroaktiven Materialaktuator, umfassend eine geschichtete elektroaktive Materialresonanzstruktur aus mindestens einer elektroaktiven Materialschicht mit einstellbarer Dicke, und eine weitere Materialschicht mit unterschiedlicher akustischer Impedanz, wobei der elektroaktive Materialaktuator angepasst ist, um ein Ultraschallsignal mit einer Frequenz vom Ultraschallwandleranordnung (30) zu empfangen, und das Ultraschallsignal an das zu behandelnde oder abzubildende Material (34) zu übertragen; und
eine Steuerung (36) zum elektrischen Steuern des elektroaktiven Materialaktuator zum Implementieren einer Resonanzfrequenzabstimmung durch Änderung der Schichtdicke durch Betätigen der mindestens einen elektroaktiven Materialschicht mit einstellbarer Dicke, um eine Verschiebung der Resonanzfrequenz bei der Übertragung des Ultraschallsignals auf das zu behandelnde oder abzubildende Material (34) zu implementieren.

**2.** Akustische Kopplungskomponente (32) nach Anspruch 1, wobei die geschichtete elektroaktive Materialresonanzstruktur mindestens zwei verschiedene Materialien (40, 42) mit unterschiedlicher akustischer Impedanz aufweist.

**3.** Akustische Kopplungskomponente (32) nach Anspruch 2, wobei ein erstes (40) der Materialien der geschichteten elektroaktiven Materialresonanzstruktur ein elektroaktives PVDF-Polymermaterial umfasst, und ein zweites (42) der Materialien der geschichteten elektroaktiven Materialresonanzstruktur ein dielektrisches elektroaktives Silikonpolymermaterial umfasst.

**4.** Akustische Kopplungskomponente (32) nach einem der vorhergehenden Ansprüche, wobei:

das elektroaktive Material zugeordnete Aktuatorelektroden aufweist, und die weitere Materialschicht eine passive Schicht ist, oder
sowohl die elektroaktive Materialschicht als auch die weitere Materialschicht elektroaktive Materialien mit jeweils zugeordneten Aktuatorelektroden sind.

**5.** Akustische Kopplungskomponente (32) nach Anspruch 4, wobei die geschichtete elektroaktive Materialresonanzstruktur eine obere (47) und untere (48) Schicht der ersten akustischen Impedanz, und eine mittlere Schicht (49) der zweiten unteren akustischen Impedanz umfasst.

**6.** Akustische Kopplungskomponente (32) nach einem der vorhergehenden Ansprüche, wobei die elektroaktive Materialschicht und/oder die weitere Materialschicht Füllstoffteilchen zum Einstellen der akustischen Impedanz umfassen.

**7.** Akustische Kopplungskomponente (32) nach Anspruch 6, wobei die Füllstoffteilchen Keramikteilchen oder Metallteilchen umfassen, die mit einer nichtleitenden Beschichtung beschichtet sind.

**8.** Akustische Kopplungskomponente (32) nach Anspruch 6 oder 7, umfassend eine segmentierte Elektrodenanordnung, die dem elektroaktivem Materialschichtaktuator zugeordnet ist.

**9.** Akustische Kopplungskomponente (32) nach einem der Ansprüche 6 bis 8, wobei der elektroaktive Materialaktuator eine elektroaktive Materialschicht mit den Partikeln und eine elektroaktive Materialschicht ohne die Partikel umfasst.

10. Akustische Kopplungskomponente (32) nach einem der vorhergehenden Ansprüche, wobei die elektroaktive Materialschicht eine elektroaktive PVDF-Polymerschicht umfasst.

11. Ultraschallvorrichtung, umfassend:

eine Wandleranordnung (30); und
eine akustische Kopplungskomponente (32), wie in einem vorhergehenden Anspruch beansprucht, die angepasst ist, um zwischen der Wandleranordnung und dem zu behandelnden oder abzubildenden Material positioniert zu sein.

12. Ultraschallvorrichtung nach Anspruch 11, wobei die akustische Kopplungskomponente (32) ein akustisch durchlässiges Fenster über der Wandleranordnung (30) ist.

13. Ultraschallvorrichtung nach Anspruch 12, wobei die akustische Kopplungskomponente (32) von der Wandleranordnung (30) beabstandet ist.

14. Ultraschallvorrichtung nach Anspruch 11, 12 oder 13, wobei die Wandleranordnung (30) angepasst ist, um Ultraschallwellen mit einer minimalen Wellenlänge in dem Material zu erzeugen, wobei die elektroaktive Materialschicht Füllstoffteilchen mit einer maximalen linearen Abmessung von weniger als 20% der minimalen Wellenlänge umfasst.

**Revendications**

1. Composant de couplage acoustique (32) destiné à être positionné entre un agencement de transducteur à ultrasons (30) et un matériau (34) à traiter ou à imager, le composant de couplage acoustique (32) comprenant:

un actionneur de matériau électroactif comprenant une structure de résonance de matériau électroactif en couches d'au moins une couche de matériau électroactif d'épaisseur réglable, et une couche supplémentaire de matériau d'impédance acoustique différente, où l'actionneur de matériau électroactif est adapté pour recevoir un signal ultrasonore ayant une fréquence de l'agencement de transducteur à ultrasons (30) et pour transmettre le signal ultrasonore au matériau (34) à traiter ou à imager; et
un dispositif de commande (36) pour commander électriquement l'actionneur de matériau électroactif pour mettre en œuvre un accord de fréquence de résonance par changement d'épaisseur de couche par l'actionnement de l'au moins une couche de matériau électroactif d'épaisseur réglable, pour mettre en œuvre un décalage de la fréquence de résonance lors de la transmission du signal ultrasonore au matériau (34) à traiter ou à imager.

2. Composant de couplage acoustique (32) selon la revendication 1, dans lequel la structure de résonance de matériau électroactif en couches comporte au moins deux matériaux différents (40, 42) d'impédance acoustique différente.

3. Composant de couplage acoustique (32) selon la revendication 2, dans lequel un premier (40) des matériaux de la structure de résonance de matériau électroactif en couches comprend un matériau polymère électroactif PVDF, et un second (42) des matériaux de la structure de résonance de matériau électroactif en couches comprend un matériau polymère électroactif diélectrique de silicone.

4. Composant de couplage acoustique (32) selon l'une quelconque des revendications précédentes, où:

le matériau électroactif comporte des électrodes d'actionnement associées et la couche de matériau supplémentaire est une couche passive, ou
à la fois la couche de matériau électroactif et la couche de matériau supplémentaire sont des matériaux électroactifs, chacun avec des électrodes d'actionnement associées.

5. Composant de couplage acoustique (32) selon la revendication 4, dans lequel la structure de résonance de matériau électroactif en couches comprend une couche supérieure (47) et inférieure (48) de première impédance acoustique, et une couche intermédiaire (49) de seconde impédance acoustique inférieure.

6. Composant de couplage acoustique (32) selon l'une quelconque des revendications précédentes, dans lequel la couche de matériau électroactif et/ou la couche de matériau supplémentaire comprennent des particules de remplissage pour régler l'impédance acoustique.

7. Composant de couplage acoustique (32) selon la revendication 6, dans lequel les particules de remplissage comprennent des particules céramiques ou des particules métalliques revêtues d'un revêtement non conducteur.

8. Composant de couplage acoustique (32) selon la revendication 6 ou 7, comprenant un agencement d'électrodes segmentées associé à l'actionneur de couches de matériau électroactif.

**9.** Composant de couplage acoustique (32) selon l'une quelconque des revendications 6 à 8, dans lequel l'actionneur de matériau électroactif comprend une couche de matériau électroactif avec le particules, et une couche de matériau électroactif sans les particules.

**10.** Composant de couplage acoustique (32) selon l'une quelconque des revendications précédentes, dans lequel la couche de matériau électroactif comprend une couche de polymère électroactif PVDF.

**11.** Appareil à ultrasons, comprenant:

un agencement de transducteur (30); et
un composant de couplage acoustique (32) selon l'une quelconque des revendications précédentes, adapté pour être positionné entre l'agencement de transducteur et le matériau à traiter ou à imager.

**12.** Appareil à ultrasons selon la revendication 11, dans lequel le composant de couplage acoustique (32) est une fenêtre acoustiquement transmissive sur l'agencement de transducteur (30).

**13.** Dispositif à ultrasons selon la revendication 12, dans lequel le composant de couplage acoustique (32) est espacé de l'agencement de transducteur (30).

**14.** Appareil à ultrasons selon la revendication 11, 12 ou 13, dans lequel l'agencement de transducteur (30) est adapté pour générer des ondes ultrasonores ayant une longueur d'onde minimale dans le matériau, où la couche de matériau électroactif comprend des particules de remplissage ayant une dimension linéaire maximale inférieure à 20% de ladite longueur d'onde minimale.

FIG. 1

FIG. 2

FIG. 3

FIG. 4

FIG. 5

FIG. 6

FIG. 7

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5638822 A
- US 5997479 A, Savord
- US 6013032 A, Savord
- US 6623432 B, Powers
- US 6283919 B, Roundhill
- US 6458083 B, Jago
- US 6443896 B, Detmer
- US 6530885 B, Entrekin

### Non-patent literature cited in the description

- Nonlinear Dielectricity of MLCCs. **S. SOMIYA.** Handbook of Advanced Ceramics: Materials, Applications, Processing, and Properties. Academic Press, 2013, 415
- **F. CARPI.** Dielectric Elastomers as Electromechanical Transducers: Fundamentals, Materials, Devices, Models and Applications of an Emerging Electroactive Polymer Technology. Elsevier, 2011, 53